# EUROPEAN PATENT SPECIFICATION

(11) **EP 2 098 206 B1**
(45) Date of publication and mention of the grant of the patent: **22.12.2010**
(21) Application number: 09002300.3
(22) Date of filing: 18.02.2009
(51) Int. Cl.: A61G 12/00, G08B 21/22

(54) **Alarm system**
Alarmsystem
Système d'alarme

(30) Priority: 03.03.2008 EP 08003893
(43) Date of publication of application: 09.09.2009
(73) Proprietor: UMIT Private Universität für Gesundheitswissenschaften Medizinische Informatik und Technik, 6060 Hall in Tirol (AT)
(72) Inventor: Hilbe, Johannes, 6020 Innsbruck (AT)
(74) Representative: Dilg, Haeusler, Schindelmann Patentanwaltsgesellschaft mbH

(56) References cited:
- EP-A- 1 417 928
- WO-A-03/096957
- NL-A- 8 701 288
- US-B1- 6 965 311

## Description

This application claims the benefit of the filing date of European Patent Application No. 08003893.8 filed March 3, 2008.

The invention relates to a device for generating an alarm upon detection of an event of a person leaving a bed.

Moreover, the invention relates to a bed.

Beyond this, the invention relates to a method of generating an alarm upon detection of an event of a person leaving a bed.

Moreover, the invention relates to a program element.

Furthermore, the invention relates to a computer-readable medium.

The care of patients increases in importance. On the one hand, it must be ensured that patients obtain all kind of services which they need from a medical and humanitarian point of view. On the other hand, a health care system must consider economical frame conditions since the budget providable by the society and the patients is limited.

However, particularly the labour-intensive care of patients suffering from diseases such as dementia is very time consuming. Particularly such kind of patients need to be monitored in a reliable manner.

NL 8,701,288 discloses a device for generating an alarm upon detection of a person leaving a bed.

US 3,781,843 discloses an apparatus for alerting nurses that a patient is attempting to leave his bed, comprising members having a fluid filled passageway therein which can be attached to the top of the side rails and foot-board of a bed. The members are coupled via lines to transducers which activate an alarm when a predetermined volume of fluid is displaced. However, a precise fluid volume measurement is difficult and prone to failure.

US 6,594,835 discloses an impact-cushioning device, which is designed to prevent injury when an occupant, confused or otherwise, falls from a height. This may be a bed or bunk, gurney or examination table. The device generally comprises an impact-reducing cushion. This cushion may be air filled or of a variety of impact absorbing materials. Also provided is a means of displacing the cushion to intercept the falling individual before he or she strikes the floor. The departure of the individual from the bed is detected by signals that are generated when one or more edge pressure switches are actuated simultaneously with signals from one or more light beam sensors. Signals thus generated are processed through an electronics module and passed to a solenoid valve. When the valve opens, gas from a canister is passed to a telescoping device. This in turn displaces the cushion. Thus a protective barrier is placed between the bed occupant and the floor. However, such a system is very complex in construction, since it relies on a sophisticated interaction between edge pressure switches and light beam sensors.

Hence, conventional alerting systems may be expensive and may lack sufficiently reliability and safety.

It is an object of the invention to provide an alarm system as claimed, which may be manufactured in a simple and cheap manner and which is reliable and safe.

In order to achieve the object defined above, a device for generating an alarm upon detection of an event of a person leaving a bed, a bed, a method of generating an alarm upon detection of an event of a person leaving a bed, a program element, and a computer-readable medium according to the independent claims are provided.

According to an example a device for generating an alarm upon detection of an event of a person leaving a bed (or a person having just left a bed) is provided, wherein the device comprises a detection unit adapted for detecting a bed edge signal indicative that a person has approached or left an edge of a bed (for instance has approached an interior edge of the bed, i.e. the person is still within the bed on a mattress and is about to leave the bed by approaching a bed rail; or an exterior edge of the bed, i.e. the person has already left a mattress of the bed, is about to leave a bed zone and has already passed a bed rail towards a protection mat which may be placed on a floor in front of the actual bed), a trigger unit adapted for triggering an alarm in case the detection unit has detected the bed edge signal indicative that the person has approached or left the edge of the bed, and a planar (for instance a basically two-dimensional structure having a large spatial extension in two horizontal orthogonal directions and a smaller spatial extension in a vertical third spatial direction) mat (for instance a mattress placed on a lying area of the bed and being surrounded by a bed rail and/or a protection mat placed on a floor along at least a part of an outer circumference of a bed rail) extending along an entire (or complete) longitudinal extension (for instance an extension along which a person lying in the bed in an ordinary manner is aligned) of a lying area (for instance a surface of a mattress of a bed on which a person using the bed is borne on) of the bed, wherein at least a part of the detection unit is embedded within (for instance is integrated within an interior of) the planar mat.

According to another example, a method of generating an alarm upon detection of an event of a person leaving a bed is provided, wherein the method comprises detecting a bed edge signal indicative that a person has approached or left an edge of a bed, triggering an alarm in case the bed edge signal has been detected indicative that the person has approached or left the edge of the bed, and providing a planar mat extending along an entire longitudinal extension of a lying area of the bed, wherein at least a part of a detection unit for detecting the bed edge signal is embedded within the planar mat (for instance a pressure-sensitive sensor probe may be entirely located within, on or below the planar mat).

According to another example, a device for generating an alarm upon detection of an event of a person leaving a bed is provided, wherein the device comprises a detection unit adapted for detecting a bed edge signal (which may be a pressure signal generated by a fluid filled lumen impacted when a person moves over a barrier such as a bed rail when the patient tries to leave the bed) indicative that a person has approached an edge of a bed, a signal duration determination unit adapted for determining whether the bed edge signal is maintained (particularly at a sufficiently high value) for at least a predefined threshold duration (i.e. for at least a minimum time), and a trigger unit adapted for triggering an alarm (particularly exclusively) in case the signal duration determination unit has determined that the bed edge signal has maintained for at least the predefined threshold duration (if this condition is not fulfilled, a very short bed edge signal resulting from artefacts such as a patient briefly touching a bed rail or the like is ignored).

According to still another example, a bed is provided which comprises at least one of the devices having the above mentioned features for generating an alarm upon detection of an event of a person leaving the bed, wherein the device is installed on or besides (for example directly besides, alongside or next to) the bed.

According to another example, a method of generating an alarm upon detection of an event of a person leaving a bed is provided, wherein the method comprises detecting a bed edge signal indicative that a person has approached an edge of a bed, determining whether the bed edge signal is maintained for at least a predefined threshold duration, and triggering an alarm in case it has been determined that the bed edge signal has maintained for at least the predefined threshold duration.

According to yet another example, a computer-readable medium (for instance a CD, a DVD, a USB stick, a floppy disk or a harddisk) is provided, in which a computer program of generating an alarm upon detection of an event of a person leaving a bed is stored which, when being executed by a processor, is adapted to control or carry out at least one of the methods having the above mentioned features.

According to still another example, a program element (for instance a software routine, in source code or in executable code) of generating an alarm upon detection of an event of a person leaving a bed is provided, which program element, when being executed by a processor, is adapted to control or carry out at least one of the methods having the above mentioned features.

Data processing for patient or infant monitoring purposes which may be performed according to examples can be realized by a computer program, that is by software, or by using one or more special electronic optimization circuits, that is in hardware, or in hybrid form, that is by means of software components and hardware components.

The term "bed edge signal" may particularly denote any signal which may be detected when a person approaches an edge of a bed (or of a bed area) from a bed internal position, that is the person is still lying on the bed and is approaching an edge of the bed so that it can be concluded that the person, when continuing the present motion, will soon leave the bed area. However, the bed edge signal may be also a signal indicative of an event that a person has already traversed an edge of the bed and is already located exteriorly of the bed so that the person has already reached an environmental spatial position around the bed. A bed edge signal captured at an exterior position of the bed in a direct surrounding of the bed can be considered as an even more reliable measure that a person leaves a bed as compared to a bed edge signal indicative of the event that the person has reached an internal edge of the bed. Under undesired circumstances, the latter can also be an artefact when the person just triggers a sensor located close to an edge of the bed but is still lying on the bed, whereas the former allows for the unambiguous and error-free conclusion that the person has actually left the bed.

The term "planar" may particularly denote a geometry according to which the mat extends along a first horizontal extension, extends along a second horizontal extension and extends along a third vertical extension being smaller than the two horizontal extensions, wherein the three mentioned extensions relate to three axes being perpendicular to one another. For instance, the vertical extension may be less than a fifth of both vertical extensions.

A "mat" may be considered as a pad of material used to space a lower surface thereof with respect to an object such as a patient placed on it. Such a mat may be made of a textile material or can be manufactured from a piece of foam which may be covered in a vinyl or plastic lining.

The term "lying area" of a bed may particularly denote a surface portion of the bed (particularly of a mattress of the bed) which is intended for being used in a manner that the person rests on this lying area during a normal use of the bed. In other words, an upper surface of a mattress of a bed may be considered as the lying area. For instance, a lying area may be defined as a convenient lying area of a person with the body height of an average-sized human being, particularly by the body height of an average-sized adult male human being (for instance having a size of 1,80m). For instance, the lying area may have a size of 90 cm x 200 cm. However, smaller or larger lengths (for instance 1,50 m to 2,50 m) and/or widths (for instance 60 cm to 1,20m) are possible as well.

The term "embedded" may particularly denote that (for instance a sensory part of) the detection unit (for instance pressure sensors, a tube filled with a fluid, etc.) may be integrated in the planar mat or may be integrally formed with the planar mat. Particularly, an outer surface of the planar mat may be free of any visual indication of the detection unit which can be buried within a textile portion of the planar mat. Such a planar mat having an embedded detection unit may be manufactured in a laminar or sandwich structure in which for instance two soft exterior layers (for instance made of foam) may enclose a detection layer which may comprise individual detection sensors or an oblong structure of a fluid filled tube. The latter may be bent along the surface of the planar mat to cover for instance basically the entire surface of the planar mat as a surface capable of sensing detection signals. For instance, a sensory portion of the detection unit may extend along the entire length and/or width of the planar mat.

According to an example, a planar mat is arranged along the full extension of a lying area of a bed having embedded therein at least a part of the detection unit. In one configuration, such a planar mat may be arranged separately from an actual mattress of the bed, wherein this planar mat may be positioned on a floor portion directly adjacent the bed. Hence, when a person such as a patient or an infant has left the bed, the weight of this person presses on a sensor integrated within the planar mat arranged in front of the bed which may serve as an unambiguous indication that the person has left the bed. Artefacts such as a false alarm can be ruled out in such an example. In addition to the detection task of such a planar mat being arranged on a floor portion in front of the bed, this planar mat may also serve as a falling protection for a person (for instance a patient suffering from dementia) having left the bed and falling on the floor. For this purpose, the planar mat may be configured mechanically soft to such an extent that the weight force of a person falling from an ordinary height of a bed (for instance 1 m or less) can be absorbed by the mat to prevent injury of the person. In such an example, the planar mat synergetically provides protection against a downfall of the person and allows for acquiring an accurate detection signal. Additionally or alternatively, the planar mat may be or may form part of an actual mattress of the bed. Also in this case, the provision of a soft lying area and a two-dimensional detection area may be synergetically combined.

When the planar mat extends along the complete lying direction of the bed, it can be ensured that a detection signal is captured from a person resting on this area or falling on such an area. Particularly in the case of a falling protection, it can be ensured that a person leaving a bed will also exert her or his weight force onto the lying area, since there is no longitudinal area at which there is no safety protection or bed leave detection.

Next, further examples of the device will be explained. However, these examples also apply to the bed, to the method of generating an alarm upon detection of an event of a person leaving a bed, to the program element and to the computer-readable medium.

In an example, the planar mat may be positioned apart from (for instance outside of) the bed so that the person leaving the bed generates the bed edge signal upon contacting the planar mat (for instance directly after having left the bed). In such an example, the planar mat may particularly be arranged on a floor adjacent to the bed. Therefore, the mattress may be arranged at a higher vertical level than the planar mat. In such a configuration, the planar mat may allow for a falling protection for a person falling from the bed on the floor. The planar mat may then mechanically absorb the falling energy and can additionally detect the event that the person has already left the bed.

The planar mat may also comprise a planar cushioning section for absorbing kinetic energy of a person falling out of the bed. For this purpose, the planar mat may have a sufficiently thick layer (for instance having a thickness of more than 5 cm) of a soft material such as foam and/or a textile material so as to be capable to prevent a person from injury when such a person (for instance an adult having an average size and weight) falls on the planar mat.

This planar cushioning section may comprise an upper cushioning layer and a lower cushioning layer, both being made of a soft material of such a material and a thickness to allow protection of the human being from injury resulting from falling out of a corresponding bed. Moreover, the detection unit may be embedded between the upper cushioning layer and the lower cushioning layer. In such a configuration, the detection unit is externally not visible or haptically perceptible so that the mat has the appearance of a normal mat. Then, the entire external surface of the mat can serve as a falling protection, and the falling detection can be performed simultaneously with the integrated detection unit.

The detection unit may comprise a tubular probe being bent once or multiple times to extend along and to cover both the longitudinal and a transversal extension of the planar mat. Therefore, the mat may extend along a lying area (which may be denoted as a longitudinal extension) and may have a significant extension perpendicular thereto which is parallel to a floor on which the planar mat is located. Then, the mat provides a two-dimensional structure serving as a falling protection and as a detection area. In order to safely detect any event of a person who has left the bed, the tubular probe may be bent (for instance in a meandrical or meander-like manner, in a zigzag manner, etc.) to cover basically the entire falling protection surface of the planar mat. This may allow to obtain a high reliability of detecting a person leaving a bed.

In an example, a detection event sensed by the detection unit may also trigger generation or enhancement of a soft falling protection. For instance, when the detection unit detects the event of a person leaving the bed, it may trigger activation of a sort of airbag. For instance, an inflatable balloon may be pumped up with a fluid such as air upon detection of an event of a person leaving the bed, thereby forming or enhancing a soft falling protection. Such an airbag may be arranged along a part or along the entire potential falling area. Multiple of such airbags may also be arranged at multiple positions so that a particular airbag to be inflated can be selected automatically based on a predicted falling position (for instance of a head or any other sensitive body portion of the person) which can be estimated automatically based on the measured detection signal(s).

Additionally or alternatively, a mattress of the bed may be provided as the planar mat or as a part thereof and may be positioned on a lying surface of the bed. Such a mattress may form the lying area of the bed so that the person leaving the bed generates the bed edge signal upon leaving the planar mat. In such a configuration, the entire lying surface is covered by a planar mat in which the detection unit may be at least partially integrated. Therefore, no additional component is necessary, since the detection unit is integrated within the mattress. Therefore, it is not necessary to provide an additional exterior component such as a sensor pad resting on a support surface of the mattress. Hence, it may be safely prevented that such a separate pad gets out of place, since a mattress may be rigidly arranged and delimited along its perimeter by a bed rail or the like.

The detection unit may comprise a tubular probe and the mattress may comprise at least one transversal recess for folding the mattress and may comprise at least one bridge element for bridging the tubular probe over the at least one transversal recess. Conventionally, a mattress, particularly a mattress for a hospital bed, may comprise one or more kink portions, for instance one or more transversal slits which allow to fold the mattress for instance to selectively adapt a shape of the mattress to a lying patient position or a sitting patient position. In order to ensure proper functioning of a tubular probe (for instance a tube filled with a fluid in order to provide for a bed leave detection) when being guided over kink recesses, bridge elements of a flexible but sufficiently rigid material may be provided at the position of the recesses which allow to bridge the tubular probe over the transversal recesses. Simultaneously folding of the mattress at the top of the transversal recess may still be allowed, thereby allowing to prevent an undesired squeezing of the tube which might otherwise interrupt fluid communication at such a kinking position. By taking such a measure, the system may be convenient to use and may safely protect against failure when kinking the mattress.

As a bridge element, it is possible to use a stretchable guide element such as a flexible but mechanically stable O profile or U profile within which the fluid tube may be mechanically guided. It is also possible to use a circumventing loop for circumventing the recess. Such a circumventing loop may extend in a basically U shape into a vertical direction of the mattress and may guide the fluid tube around a recess.

The device may (additionally or alternatively to the planar mat) further comprise a signal duration determination unit adapted for determining whether the bed edge signal is maintained for at least a predefined threshold duration. The trigger unit may be adapted for triggering an alarm in case the signal duration determination unit has determined that the bed edge signal has maintained for at least the predefined threshold duration. According to such an example, an alarm generation system may be provided which detects the event of a person (such as a patient or an infant) leaving a bed, and in this context generates a bed edge signal. Such a signal may be any signal which is indicative of the trial of a person to move away from a bed. For example, a pressure detector, a photoelectric barrier sensor, a touch sensor, an electronic system comprising two spaced conductors being brought in contact upon exertion of pressure or the like may be implemented for this purpose. However, according to an example, the event of a person leaving a bed is only considered by the system when the bed edge signal is maintained (particularly without interruptions) at least for a minimum time (for instance several seconds) which can be defined by an operator of the system or which can be fixed at a factory side. By triggering an alarm only in case that the bed edge signal maintains for more than a minimum time period, it can be safely prevented that a false alarm is generated in the absence of the event of a person trying to leave a bed. For instance, when a person simply touches a rail of a bed, a short bed edge signal (such as a brief pressure pulse) may be generated which nevertheless is not a reliable indicator that the person indeed tries to leave the bed. Since such artefacts are usually very short in time, the time criteria implemented according to an example of the invention has turned out to be a very secure indicator allowing to automatically distinguish a bed leave trial from other events.

Thus, examples provide a system for preventing a fall of a patient or an infant, for instance in a hospital environment or in a home environment. For that purpose, a warning system may be provided which informs a destination person (such as nursing staff in a hospital or in a foster home, or parents of an infant), in case that a person such as a patient being prone to fall tries to leave a bed. According to an example, such a warning system may comprise pressure sensitive elements which may be fastened at an edge of the bed (preferable at bars of the bed). The pressure sensitive elements may comprise a gas tube system (preferable air) coupled to a pressure sensor in which a change of the gas pressure can be converted into an electrical signal or the like. The gas pressure change may be the result of a compression of the tube, resulting from a trial of a patient to move over the edge of the bed, or to try to leave the bed in another manner. The generated signal may be integrated in a bell system of a hospital ward. Thus, by such an alarm nurse staff may be informed in time that a person is leaving the bed so that a fall-out of the bed can be prevented or the person can be guided back to the bed after the alarm has been activated.

The planar mat (in an example in which it forms the bed mattress or in an example in which it forms the falling protection mat) may comprise a mat body (for instance a main body of the planar mat comprising cushioning or padding) and a mat cover (such as a fitted sheet, for example a textile material which may be made of a watertight fabric) stretchably mounted over at least a part (for instance covering the upper part, wherein at least a portion of a lower part may be free of the mat cover) of the planar mat. At least a part of the detection unit (for instance a detection probe in the form of a pressure-sensitive fluid filled tube) may be integrated in a thin sheet-like mat cover. For instance, a pressure-sensitive probe may be included or worked in at an inner surface of the mat cover. In an example, it is possible that a tubular textile member is fastened (for instance adhered or sewn) to an inner surface of the mat cover and the pressure-sensitive probe (such as a fluid-filled tube) is arranged within such a tubular textile member. This may allow to obtain a stretchable configuration with sufficient stability. A separate member for protecting a tubular probe against kinks (of the mat body) may be omitted in such an embodiment. Further, the detection sensitivity of such an embodiment may be very accurate.

The detection unit may be adapted for detecting whether the bed edge signal has an amplitude of at least a predefined threshold amplitude, and the trigger unit may be adapted for triggering an alarm (particularly exclusively) in case that the detection unit has detected that the bed edge signal has an amplitude of at least the predefined threshold amplitude. Thus, according to this embodiment, only in case of a logical "AND" combination of a minimum amplitude such as a minimum pressure and simultaneously a minimum duration of the signal triggers an alarm which has turned out to further reduce the risk of false alarms which do not result from a patient leaving the bed.

The detection unit may comprise a probe having one or more lumen and may comprise a pressure sensor (for instance may have exactly one pressure sensor) adapted for measuring a pressure of a fluid tightly sealed within the lumen which fluid is impactable by an event of a person leaving a bed, thereby actuating the probe resulting in a fluid displacement. A single pressure sensor may be sufficient for that purpose which may be coupled to an end portion of the lumen and, in response to a pressure pulse or signal resulting from a person actuating the probe when trying to leave the bed, generates a corresponding signal. When a person tries to leave the bed, she or he will automatically apply a pressure on the probe generating a pressure signal indicative of the event. Alternatively, multiple pressure sensors may be implemented.

The lumen of the probe may comprise a fluid. Such a fluid may comprise a liquid such as water. Alternatively, such a fluid may comprise a gas like air, oxygen, hydrogen, nitrogen, helium, etc. When the lumen is pressure-sealed or pressure-tight, even quite small forces acting on the probe may result in a pressure signal resulting from a displacement of the fluid, allowing to provide a system with high accuracy. The device may use a tank comprising fluid material or may include only the fluid material within the lumen. The latter example allows for a small dimensioned device and nevertheless provides a high degree of accuracy.

The probe may be adapted to be mountable on a bed, particularly on a rail of a bed. Thus, when a person tries to leave the bed and passes the rail, the weight force of the person acting on the probe is converted into a pressure signal or the like which is a reliable indicator that the person tries to leave the bed.

Examples may be applied to a bed having separated or divided rails or bars.

The probe may comprise an attachment section adapted for being attachable to a bar (or a rail) of a bed. For example, such an attachment section may be shaped and/or dimensioned in a manner to correspond to a shape of a bar of a bed so that, when the attachment section is attached to the bar of the bed, the probe securely rests on the bar. Such an attachment section may be made of a rubber material or of any kind of plastic material. It may have a protrusion (or recess) cooperating with a corresponding recess (or protrusion) on the bar of the bed. Alternatively, the attachment section may have an engagement portion engaging an upper portion of the bar of the bed. Further alternatively, the attachment section may comprise a clip fastener adapted for fastening a probe or sensor at a bar or rail of a bed by clipping on the probe or sensor at the bar or rail of the bed. This allows for a fast and easy connection.

The probe may comprise an attachment section having a recess for receiving a bar (or a rail) of a bed. When the attachment section is mounted on the bar, a positive locking force and/or friction may keep the attachment section fixed on the bar.

The probe may comprise a flexible housing section providing for an external boundary of the probe when being mounted on a bar (or a rail) of a bed. Such a flexible housing portion may be made, for instance, from neoprene which is a flexible and nevertheless stable and robust material which allows to use the bed in any desired environment.

The probe may comprise a fastener section adapted for fastening the probe on a bar (or a rail) of a bed. By such a fastener section, it can be ensured that the probe is permanently installed on a bed that cannot be easily removed, for instance by a patient.

Preferably, such a probe comprises a zip fastener so that by operating a zip mechanism, the probe can be easily attached to the bar or can be removed from the bar, for instance for installation, repair or maintenance. With a zip fastener section, a single hand movement may be sufficient to fasten or unfasten the fastener section on a bar of a bed. When such a zip fastener is provided on a bottom surface of the bar (or a rail), it is not visible from outside and is therefore not prone to misuse. Such an example allows to retrofit a conventional bed with a sensor system according to an example.

The probe may be integrally formed with a bar (or a rail) of a bed. Such a configuration may be appropriate when constructing a new bed having a sensor system according to an example integrated undetachably in the bed.

The probe may comprise a flexible member (such as a tube, for instance a flexible tube) surrounding the one or more lumen, which member may be arranged between the attachment section and the flexible housing section. This flexible tube accommodating the lumen may be compressed by the weight force exerted by a person trying to leave the bed and should therefore be made of a material having a sufficient flexibility to allow to be compressed under the impact of a typical weight of a person. On the other hand, the flexible tube should allow a gas under a pressure of, for instance 1bar, to fill the lumen so that the exertion of a force on a top of the probe is converted into a force acting on the tube, compressing the fluid included therein, resulting in a pressure signal. A cellular rubber, foam rubber or sponge rubber may be preferred material for the flexible member.

The signal duration determination unit may comprise a delay for preventing the bed edge signal from being forwarded to the trigger unit until the predefined threshold duration has expired. Thus, such a delay unit which can be an electronic or mechanical element delays supply of the bed edge signal to the trigger unit for the predefined threshold duration, thereby ensuring that no false alarm is generated in case that a person only temporarily hits or compresses the probe without trying to leave the bed. The provision of a delay is a simple but very efficient mechanism to provide this function.

The device may comprise an alarm generation unit adapted for generating (and optionally outputting) a perceivable alarm signal when the trigger unit has triggered an alarm. Thus, upon activating the trigger unit, an alarm signal may be generated which can be perceived by a human being such as a nurse or parents.

Such an alarm signal can be an acoustic alarm signal, such as a bell or an electric horn. Additionally or alternatively, an optical alarm signal may be generated such as a light flash or the activation of a, for instance red, lamp. Also a haptic alarm signal may be triggered, for example a vibration alarm of a mobile phone or any other portable unit carried by an operator of the device allowing to use the handheld device at a remote location from the bed to monitor a person from a remote position. Of course, such a handheld device may also include an acoustic and/or an optical alarm signal generator. Other kinds of alarms are possible as well, for instance the generation of an SMS, an MMS or an e-mail when the alarm is detected.

The predefined threshold duration (which, in an example, has to be exceeded to trigger an alarm) may be in a range between 1s (or about 1s) and 10s (or about 10s), particularly in a range between 2s (or about 2s) and 5s (or about 5s). These parameters have turned out to be reliable parameters indicative of typical actions of a patient leaving a bed.

The predefined threshold amplitude (which, in an example, has to be exceeded to trigger an alarm) may be in a range between 1mbar (or about 1mbar) and 10mbar (or about 100mbar), particularly in a range between 5mbar (or about 5mbar) and 20mbar (or about 20mbar).

The device may further comprise a wireless transmission unit (such as an antenna system) adapted for transmitting an alarm signal generated by the triggering unit to a remote communication partner apparatus in a wireless manner. Therefore, when the device has triggered an alarm, this alarm does not necessarily have to be supplied via a wired connection to a destination, but can be also transported in a wireless manner, for example via a mobile communication network, by Bluetooth, by infrared transmission, etc. This allows to use a handheld device or any other communication partner apparatus at the remote position from the bed to be monitored, so that a remotely located person can monitor a patient or a child in the bed without being present in the room. However, it is also possible to transmit the alarm signal in a wired manner, for instance as a current or voltage signal.

The device may comprise the remote communication partner apparatus adapted for communicating with the wireless transmission unit in a wireless manner and adapted for outputting an alarm upon receipt of the alarm signal. The communication between communication partner apparatus and wireless transmission unit may be unidirectional or bidirectional. Such a communication partner apparatus may be a mobile phone, a remote control, a personal digital assistant (PDA), an MP3 player, or any other handheld device.

The device and the one or more communication partner apparatuses may form a communication system.

The device may be a hospital patient monitoring device. Thus, a patient in a hospital may be monitored automatically and, upon detection that the patient leaves the bed, a nurse may rapidly move to the bed of the patient to guide the patient back in the bed or to prevent the person from leaving the bed or falling. Moreover, a medical or humanitarian service can be provided rapidly to the patient which improves the quality of the care.

Another field of application of examples is a home care patient monitoring device which allows a, for instance ill, person at home to be monitored by a relative or by a nurse.

Another example is an infant monitoring device, i.e. some kind of baby monitor which allows to monitor a baby in a bed from a remote position such as another room in a house or even apart from a house. Such a system may be preferred over conventional baby monitoring systems, since baby monitoring systems only relying on acoustic signals of a baby suffer from the shortcoming that when the baby leaves the bed and therefore the sensitive range of the microphone of such an acoustic baby monitoring system is left, no reliable signal can be generated. In contrast to this, according to an example, when a baby leaves a bed, she or he has to move definitely over the barrier at an edge of the bed, therefore automatically activating the alarm system. The detection unit may comprise two spaced conductors being brought in contact to generate the bed edge signal upon exertion of pressure by the person when approaching the edge of the bed.

Examples can be combined with an infrared light barrier system or with an electrical system generating a current signal upon detection of a person leaving the bed. Thus, a redundant system may be provided which combines the advantages of the different detection principles, thereby further increasing the reliability. For instance, a baby monitoring system may combine an alarm system with a conventional acoustic sensing system detecting acoustic signals of a baby, thereby allowing for a more reliable indication that a baby needs help.

Particularly patients suffering from Alzheimer's disease or dementia may be let alone in a room and can be monitored by the reliable bed leaving system, which allows to properly care for such patients while eliminating the necessity to permanently rest at the bed of the patient. By providing such a safety device at the bed of a patient and implementing a pressure sensorics, not only a simple yes/no characteristic may be measured, but a more detailed basis of information may be measured for reliably distinguishing between events such as a person putting the hand on rail of the bed and a person leaving the bed. According to an example, also the time dependence of the pressure signal can be evaluated, and a pattern of a time dependence of the pressure signal can be compared to a stored threshold pattern which is typical for a person leaving a bed. Therefore, the reliability of the system can be further increased. In such a context, it is also possible to consider the spatial dependence of pressure signals generated and measured at different positions of a boundary of a bed, and allowing to distinguish (for instance involving pattern analysis) between a bed leave event and other events.

The cumulative fulfilment of a required minimum time and required minimum load allows to reduce false alarms. Such a pressure signal may be forwarded to a time delay, which only triggers an alarm when the signal lasts for, for instance, 2 to 5 seconds. Thus, examples may prevent false alarms as well as provide for an improved compliance.

For example, a compression of a tube may generate a pressure in a millibar range, which can be conducted via a pressure-tight plastic tube to a pressure transducer, which supplies the signal further to a time switch logic.

The aspects defined above and further aspects are apparent from the examples to be described hereinafter and are explained with reference to these examples.

The invention will be described in more detail hereinafter with reference to examples of embodiment but to which the invention is not limited.
Fig. 1 illustrates a bed having mounted thereon a device for generating an alarm upon detection of an event of a person leaving the bed.
Fig. 2 is a cross-sectional view of a bar of a bed and a probe of the device of Fig. 1 mounted on the bar.
Fig. 3 illustrates a tube member of a device.
Fig. 4 illustrates a flexible housing section of a probe of a device.
Fig. 5 illustrates electronics of a device.
Fig. 6 illustrates a bed integrally formed with a device for generating an alarm upon detection of an event of a person leaving the bed.
Fig. 7 is a cross-sectional view of a bar of a bed and a probe of the device of Fig. 6 integrally formed with the bar.
Fig. 8 is a flow chart illustrating a method.
Fig. 9 illustrates a communication system having a device and a mobile communication unit.
Fig. 10 to Fig. 12 each illustrate a bed having mounted thereon or adjacent thereto a device for generating an alarm upon detection of an event of a person leaving the bed.

The illustration in the drawing is schematically. In different drawings, similar or identical elements are provided with the same reference signs.

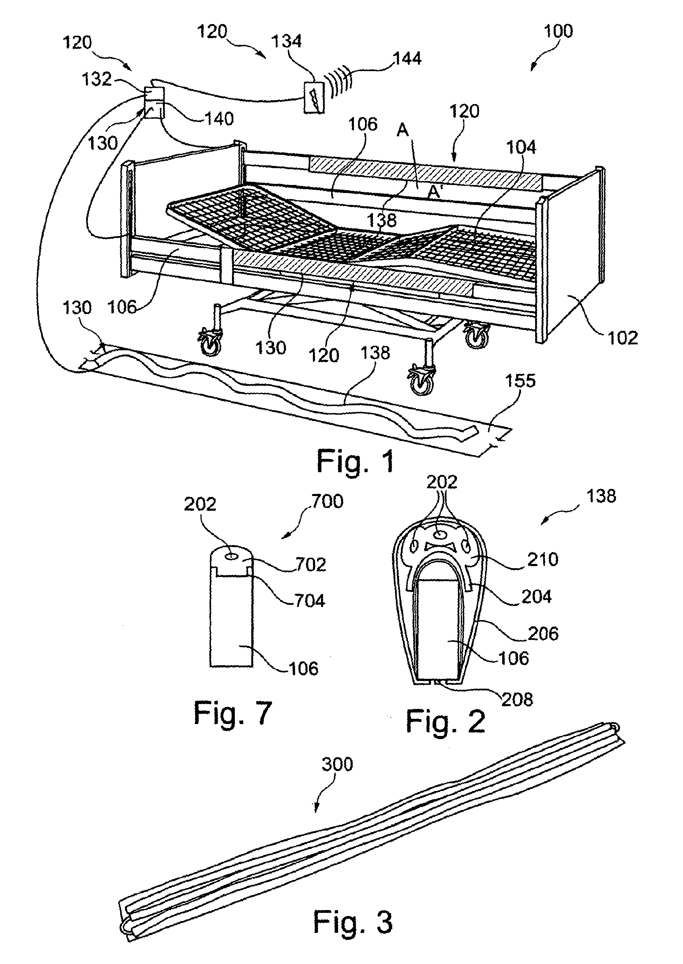

**Fig. 1** illustrates a bed 100. The bed comprises a support 102, a lay-down space 104 and parallel aligned rails 106 which may also be denoted as bars and which laterally limit the bed 100.

The bed 100 comprises a device 120 for generating an alarm upon detection of an event of a person (not shown in the figure) lying in the bed 100 and trying to leave or having already left the bed 100, which device 120 is installed on the bed 100, as will be described in the following.

The device 120 comprises a detection unit 130 for detecting a bed edge signal indicative that a person has approached an edge 106 of the bed 100 or has already left the bed 100. Furthermore, a signal duration determination unit 132 is provided which is coupled with the detection unit 130 and which is adapted for determining whether the bed edge signal is maintained for at least a predefined threshold duration.

Moreover, a trigger unit 134 is provided which is coupled with the signal duration determination unit 132 and is adapted for triggering an alarm such as an acoustical alarm 144 in case the signal duration determination unit 132 had determined that the bed edge signal has maintained for at least the predefined threshold duration.

More particularly, the detection unit 130 is adapted for detecting whether the bed edge signal has a pressure value of at least a predefined threshold pressure. The trigger unit 134 is adapted for triggering an alarm only in case the detection unit 130 has detected that the bed edge signal has a pressure value of at least the predefined threshold pressure.

A probe 138 is mounted on the bed 100, particularly on the rails 106 of the bed 100.

Fig. 1 further shows a planar mat 155 extending along an entire longitudinal extension of a lying area of the bed 100, wherein the probe 138 of the detection unit 130 is embedded also within the planar mat 155. The planar mat 155 serves synergetically for detecting a bed edge signal (when the person having left the bed stands on the planar mat 155 or falls on the planar mat 155) and as a soft falling protection (when the person having left the bed falls on the planar mat 155) for absorbing falling energy to prevent injury of the person.

The detection unit 130 comprises the probe 138 which is shown in more detail in **Fig. 2** and which has several lumen 202 and comprises a pressure sensor 140 adapted for measuring a pressure of a gas within the lumen 202 which gas is impactable by an event of a person trying to leave the bed 100. The cross-sectional view of the probe 138 in Fig. 2 is taken along a line A-A' of Fig. 1.

The lumen 202 of the probe 138 comprises air as the gas. This air is accommodated within the lumen 202 in a gas-tight manner.

The probe 138, as shown in Fig. 2, comprises an attachment section 204 attached to the bar 106 of the bed 100. A flexible housing section 206 made of neoprene is provided as an outer circumference of the probe 138 for providing an external boundary of the probe 138 when being mounted on the bar 106 of the bed 100. The probe 138 furthermore includes a fastener section 208, namely a zip fastener section 208, for detachably fastening the probe 138 on the bar 106 of the bed 100. The probe 138 comprises a flexible tube 210 made of a foam rubber and surrounding the lumen 202, which tube 210 is arranged between the attachment section 204 and the flexible housing section 206. When a person tries to leave the bed 100, she or he has to move over the rails 106, thereby applying a weight force to the probe 138, thereby compressing tube 210 and therefore displacing gas in the lumen 202, thus generating a pressure signal detectable by the pressure sensor 140.

The plastic profile 210 is made of an air-tight material. Such a hollow profile 210 generates the pressure signal detected by the pressure transducer 140. For fastening the attachment section 204, a groove may be milled, and the rubber item may be inlaid therein. The neoprene material 206 is an anti-slip material which prevents the probe 138 from changing position upon exertion of a weight by a person on the probe 138. The zip fastener 208 is particularly appropriate for fastening the probe 138 at a chrome steel post.

It is possible to integrate the probe 138 shown in Fig. 2 in the planar mat 155. Alternatively, it is also possible that only a soft fluid-filled tube is integrated in the planar mat 155.

Now, reference is made again to Fig. 1. A pressure signal generated by a person may be converted via the pressure sensor 140 into an electronic signal which can be maintained, by the signal duration determination unit 132, for a predefined time. More particularly, the signal duration determination unit 132 comprises a delay for delaying the bed edge signal from being forwarded from the pressure sensor 140 to the trigger unit 134 for a predefined threshold duration of, for instance, several seconds. When the trigger unit 134 has triggered an alarm, the acoustical signal 144 is generated perceivable by a human being such as a nurse in a hospital environment.

A voltage supply of the alarm unit 134 may be provided as well as a signal transmission via a nurse call system.

**Fig. 3** shows a detailed view of reference numerals 204, 210 of Fig. 2.

The material used for the component shown in image 300 of Fig. 3 should be stable in shape, but sufficiently compressible. It may advantageous that the material can be fastened by adhering. For example, a foam rubber ("Moosgummi") is preferred. The signal generation may be achieved by a compression of the material 210 which, in turn, compresses the one or more lumen 202. Thus, an increased air pressure can be generated.

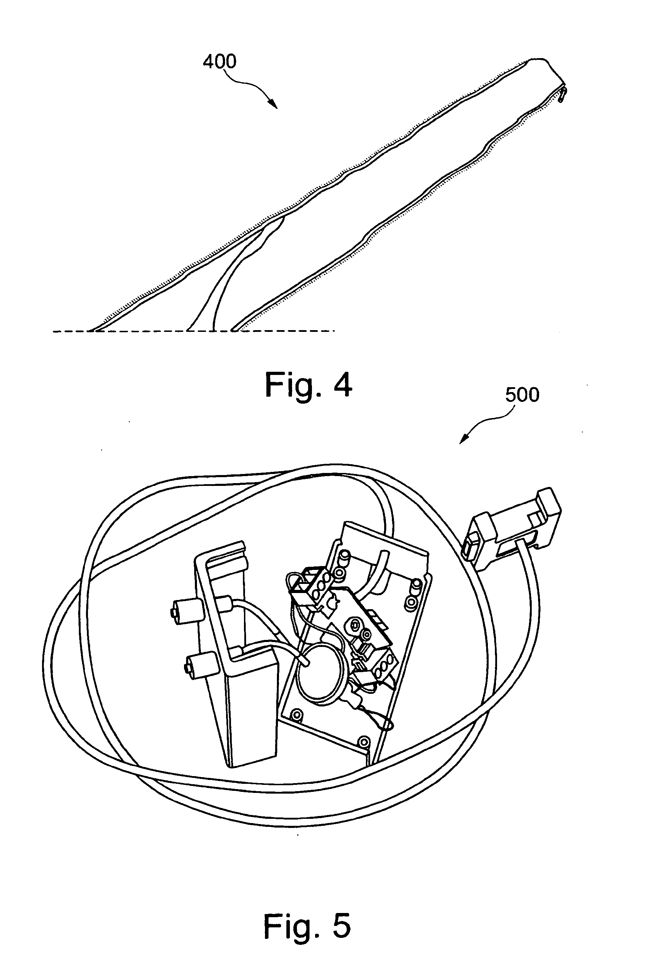

**Fig. 4** is an image illustrating a detailed view of component 206 and 208 of the probe 138 of Fig. 2. The material of the member 206, neoprene in the present example, should be expendable at an exterior boundary, should be easy in maintenance and should have a high robustness regarding mechanical influences. The interior surface should additionally also have a sufficiently high friction at the surface. Regarding the signal generation, the components serve for fixing the sensoric on the upper surface of the bar 106. Both components may be connected by the zip fastener 208.

An electronics member 500 shown in **Fig. 5** serves for signal generation, signal conversion and a time delay may be provided in order to delay the signal for a proper alarm/false alarm ratio for a predefined time.

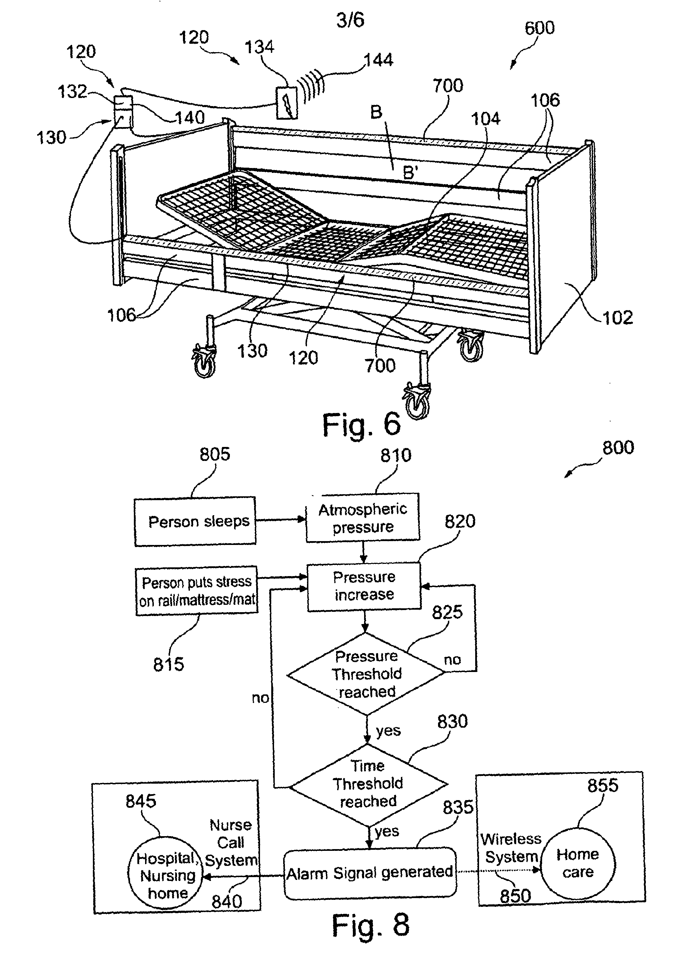

**Fig. 6** shows a bed 600, which differs from the bed 100 shown in Fig. 1 in that the components of the device 120 are integrated in the bed 600, and not retrofitted.

Although not shown in Fig. 6, it is possible to implement a planar mat 155 in this example. For the configuration of such a planar mat 155, reference is made to Fig. 1, Fig. 10 to Fig. 12.

In the bed 600, the probe is configured as the probe 700 shown in **Fig. 7****.**

The probe 700 has a single lumen 202 within a hollow plastic profile 702 which can be made of foam rubber. A post 704 at a bottom portion of the hollow profile 702 is shaped to correspond to a recess on an upper surface of the bar 106 where the hollow profile 702 is fastened, for instance adhered.

**Fig. 8** shows a flow chart 800 illustrating a process.

When a person sleeps in a bed, see block 805, the pressure signal detected by the pressure sensor 140 is the atmospheric pressure, see block 810. When a person puts stress on a rail 106 and/or on a mattress 1024 and/or on a planar mat 155 since the person tries to leave the bed 100, 600, see block 815, a pressure increase, see block 820, is applied on the pressure sensor 140. In a block 825 it is checked whether a predefined pressure threshold has been reached by the pressure signal. If no, the system goes back to block 820. If yes, it is checked in a block 830 whether the time threshold is reached, i.e. whether the pressure signal exceeding the pressure threshold lasts for more than a predefined threshold pressure. If no, the system goes back to block 820. If also the time threshold is reached, an alarm signal is generated, see block 835.

When an alarm signal is generated, this alarm can be transmitted via a nurse call system 840 of a hospital or a nursing home 845.

In a wireless system, see reference numeral 850, such a signal may be transmitted to a home care block 855.

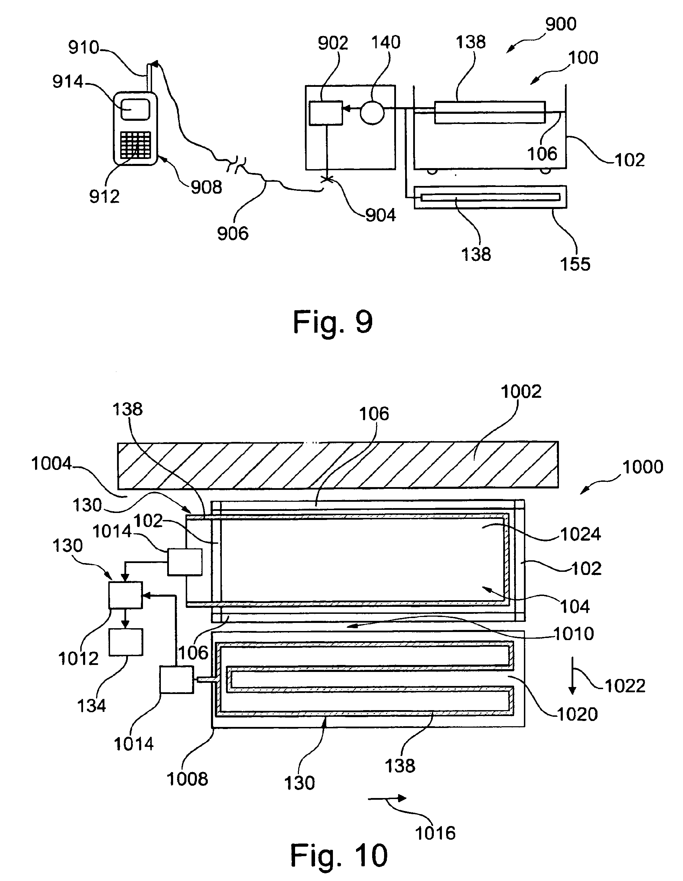

**Fig. 9** shows a communication system 900.

A bed 100 as the one shown in Fig. 1 is illustrated schematically.

When a person (not shown in Fig. 9) leaves the bed 100, a pressure signal is supplied from the probe 138 to the pressure sensor 140. The pressure sensor 140 may supply the signal to a control unit 902 such as a central processing unit (CPU) or a microprocessor. When the control unit 902 has detected an alarm, the alarm signal may be supplied to a wireless transmission unit 904 such as an antenna for transmitting a wireless alarm signal 906 by the triggering unit 904 coupled to the control unit 902 to a remote communication partner apparatus, namely a handheld device 908 having an antenna 910 in a wireless manner. The portable unit 908 is adapted for communicating with the wireless transmission unit 904 in a wireless manner and can output an alarm (for instance an acoustic, an optic and/or a haptic alarm) upon receipt of the alarm signal 906. For example, parents of a child carrying the portable device 908 can be informed that the child at home leaves the bed 100.

The portable unit 908 comprises a user interface for enabling a user to bidirectionally communicate with the portable unit 908. Thus, the user may supply the portable unit 908 via the user interface with control demands via input elements 912 such as buttons, a keypad, a joystick, or the like. Furthermore, an alarm may be displayed to a user on a display unit 914 of the user interface, for instance a liquid crystal display (LCD) unit or the like.

Further, Fig. 9 schematically shows a planar mat 155 extending along an entire longitudinal extension of a lying area of the bed 100, wherein the probe 138 of the detection unit 130 is embedded within the planar mat 155 and is functionally connected with pressure sensor 140.

**Fig. 10** shows a bed 1000.

Fig. 10 shows a plan view of the bed 1000 positioned in a room which is delimited by a wall 1002. Support 102, lay-down space 104 and parallel aligned rails 106 of the bed 1000 can be seen in Fig. 10 as well and can be configured, for instance, as shown in Fig. 1. The bed 1000 rests on a floor 1004 on which also a planar mat 1008 is directly positioned on.

The bed 1000 is capable of generating an alarm upon detection of an event of a person (not shown) leaving the bed 1000. A detection unit 130 is provided for detecting a bed edge signal indicative that the person has approached or even left an edge 1010 of the bed 1000. The detection unit 120 comprises a control unit 1012 which receives detection signals from each of two tubular probes 138 each of which being coupled with a corresponding one of two pressure sensors 1014. When the control unit 1012 receives a signal from one of the pressure detectors 1014, the control unit 1012 can conclude that the person has left the edge 1010 of the bed 1000 and can therefore send a corresponding signal to a trigger unit 134 adapted for triggering an alarm such as an audible alarm.

In the following, the detection characteristic of the embodiment of Fig. 10 will be explained in more detail.

In the example of Fig. 10, a first planar mat 1008 is provided in front of the rail 106 which extends along an entire longitudinal extension 1016 of lying area 104 (indicated in Fig. 10 as a hatched area) of the bed 1000. As can be taken from Fig. 10, tubular probe 138 is embedded within the first planar mat 1008. Fig. 10 further shows that the first planar mat 1008 is positioned apart from the bed 1000 so that the person leaving the bed 1000 generates the bed edge signal upon contacting the first planar mat 1008. In other words, the first planar mat 1008 is directly placed on floor 1004 in the room shown in Fig. 10. The first planar mat 1008 comprises a planar cushioning section 1020 made of a soft material such as a foam and therefore serves as a falling protection for protecting a person falling out of the bed 1000 onto the first planar mat 1008 positioned on the floor 1004. As can be further taken from Fig. 10, the tubular probe 138 integrated within the first planar mat 1008 is bent in a meander-like manner so as to extend along and to cover both the longitudinal direction 1016 and a transversal direction 1022 of the first planar mat 1008.

Additionally, a second planar mat 1024 is provided as a mattress 1024 on the bed 1000 forming the lying area 104 of the bed 1000 so that the person leaving the bed 1000 generates the bed edge signal upon leaving the second planar mat 1024. As can be taken from Fig. 10, further tubular probe 138 extends within the second planar mat 1024 first along the longitudinal direction 1016, is then bent to be aligned along the transversal direction 1022 and is bent again to extend along longitudinal section 1016. Therefore, the probe 138 is aligned parallel to the bed rails 106 and to the support 102. Other geometries of this probe 138 of the mattress 1024 are possible, for instance a meander-shape or a zig-zag shape.

Therefore, both probes 138 integrated within the first planar mat 1008 and within the second planar mat 1024 are capable of providing a separate pressure signal to be detected by the assigned pressure detector 1014 when the person either presses on the fluid filled probe 138 of the second planar mat 1024 when being about to leave the bed 1000 or presses against the tubular probe 138 of the first planar mat 1008 after having left the bed 1000. The occurrence of one of these signals or the coincidence of both signals may be defined as a decision criteria that an alarm is triggered indicating that the person has left the bed 1000. The control unit 1012 can receive two independent signals from the pressure sensors 1014 so that the alarm of the trigger unit 138 can be triggered in a very reliable manner.

Although Fig. 10 shows an example comprising two different planar mats 1008, 1024, one of the two mats 1008, 1024 can be omitted in another example, for instance to obtain a more simplified configuration.

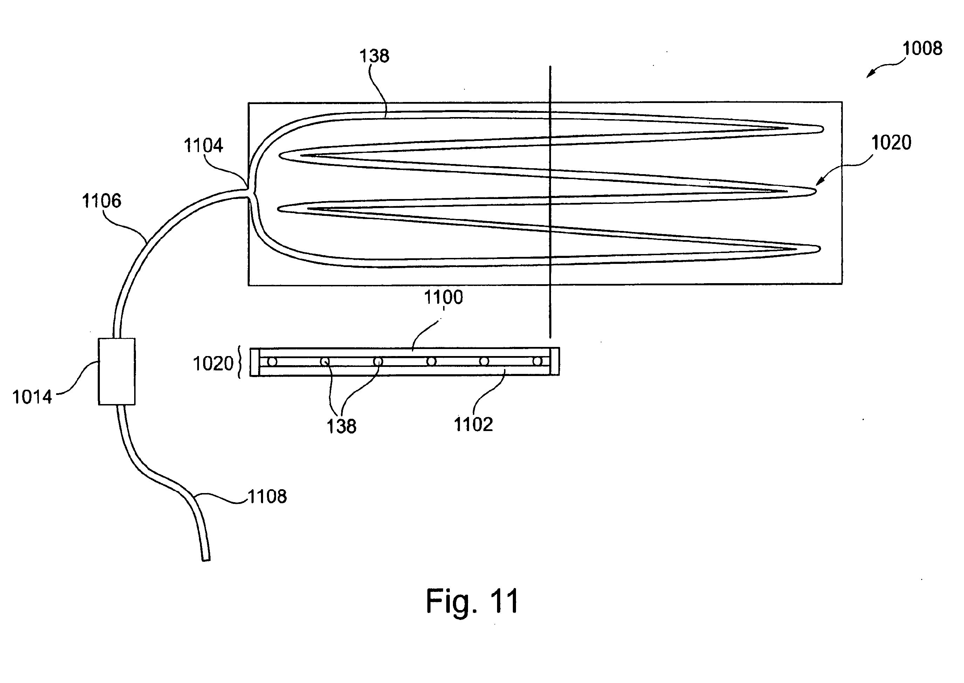

**Fig. 11** shows a plan view and a cross-sectional view of a planar mat 1008 similar to the one implemented in the example of Fig. 10.

As can be taken from Fig. 11, the cushioning section 1020 of the planar mat 1008 (shown also in Fig. 10) comprises an upper cushioning layer 1100 and a lower cushioning layer 1102, wherein the probe 138 of the detection unit 130 is embedded between the upper cushioning layer 1100 and the lower cushioning layer 1102. As can be further taken from Fig. 11, the tubular probe 138 comprises a T-splitter 1104 for providing fluid communication between a connection portion 1106 of the tube and a bent portion of the probe 138. Therefore, it is sufficient to provide a single pressure sensor 1014 for operating the entire hollow tube 138, 1106 of mat 1008. By the bent configuration of the probe 138, basically the entire two-dimensional surface of the downfall protection mat 1008 is sensitive regarding pressure exerted by the weight force of a patient falling out of the bed. Therefore, the event of a person leaving the bed can be safely detected. Moreover, the mechanical damping function of the cushioning layers 1100, 1102 in combination with the dampening function of the fluid filled tube 138 allows a person to be prevented from injury when falling out of the bed.

An electrical signal indicative of the detection result can be passed via an electrical connection line 1108 to a connected entity such as a control unit 1012. The cushioning layers 1100, 1102 are made of rubber foam. With the fall protection mat 1008, it is possible that nursing staff in a hospital (or parents of an infant) can be informed in a simple and reliable way when a patient prone to falling out of a bed leaves the bed. For this purpose, a padding in the form of the cushioning layers 1100, 1102 is provided. As can be taken from Fig. 11, this padding can be formed from at least two layers 1100, 1102. Sandwiched between these layers 1100, 1102 is the tubular probe 138 (in a similar manner as in case of a floor heating). Pressure-tight conduits 138, 1106 are connected to the pressure switch 1014. When a person falls out of the bed, the collision energy can be distributed over a large area and can simultaneously generate a pressure within the closed pneumatic or hydraulic system, so that an alarm can be triggered based on an electric signal generated by the pressure switch 1014 and conducted via the electric connection line 1108. Also in a scenario in which a person moves out of a bed without falling and puts her or his feet on the sensor cushioning mat 1008, it is also possible that a sensor signal is triggered by the above-described scheme to inform nursing staff or parents.

In Fig. 11, the probe 138 can be provided in a similar manner or identical manner as described above referring to Fig. 2 or Fig. 7.

However, in this configuration it is specifically possible that a profile (for instance made of foamed rubber) is used having an interior lumen which is sealed. Via the pressure tight line or conduit 138, 1106, the pressure sensor 1014 can be connected. When the lumen is squeezed by an external impact, an increased pressure is generated. This can trigger an electric signal via electric pressure switch 1014.

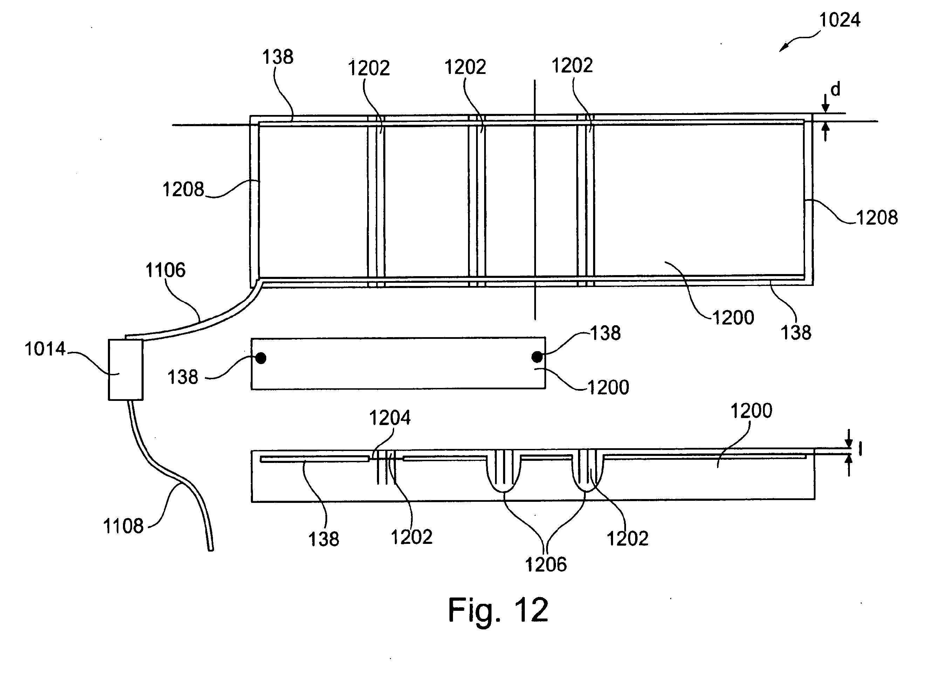

Next, referring to **Fig. 12****,** a mattress 1024 of the type shown in Fig. 10 will be described in more detail.

In the example of Fig. 12, the detection unit comprises a tubular probe 138 integrated within a foam rubber material 1200 of the mattress 1024.

As can further be taken from Fig. 12, the mattress 1024 comprises a plurality of transversal recesses 1202 (transversal with respect to an ordinary lying direction of a person lying on mattress 1024). The recesses 1202 are slits drilled in the rubber foam material 1200 of the mattress 1024. A bridge element 1204 bridges transversal recess 1202 and can be configured as a bendable but sufficiently stable material. Other recesses 1202 may be bridged by circumventing loops 1206 which circumvent the respective recess 1202, as can be taken from Fig. 12 as well. Furthermore, connection lines 1208 are shown in Fig. 12.

With the configuration of Fig. 12, it is possible to inform a nursing staff in a hospital (or parents of an infant) in a simple way when a patient being prone to falling out of a bed (or an infant) tries to move out of the bed. For this purpose, in the example of Fig. 12, a tubular probe 138 of the type described above can be worked in the rubber foam material 1200 of the mattress 1024 over the entire length of the lying area 104 or over a part thereof. For instance, the tubular probe 138 may be connected to the rubber foam material 1200 of the mattress 1024 using an adhering technique. In an example, a distance "d" between the rubber tube 138 and a side edge of the mattress 1024 can be in a range between 1 cm and 5 cm. It may be embedded for instance 1 cm to 2 cm deep (see distance "I") in the mattress 1024. The recesses 1202 are bridged by means of the loops 1206 or the bendable members 1204. When a person tries to move out of the bed, a pressure signal may be generated in the closed hydraulic or pneumatic system 138, 1106. When this pressure exceeds a predefined threshold, a pressure switch 1014 sends a corresponding signal to an alarm system via an electric connection line 1108. The pressure switch 1114 can be adjusted in such a manner as to achieve a desired ratio between alarms and false alarms.

According to different examples, various configurations are possible: detection only at a rail, detection only on a mattress of a bed, detection only on a mat arranged on a floor adjacent to the bed, a combination of two of these measures, and a combination of all three of these measures.

It should be noted that the term "comprising" does not exclude other elements or features and the "a" or "an" does not exclude a plurality. Also elements described in association with different examples may be combined.

It should also be noted that reference signs in the claims shall not be construed as limiting the scope of the claims.

## Claims

1. A device for generating an alarm upon detection of an event of a person leaving a bed (1000), wherein the device comprises
a detection unit (130) adapted for detecting a bed edge signal indicative that a person has approached or left an edge of a bed (1000);
a trigger unit (134) adapted for triggering an alarm in case the detection unit (130) has detected the bed edge signal indicative that the person has approached or left the edge of the bed (1000);
a planar mat extending along an entire longitudinal extension of a lying area of the bed (1000), wherein at least a part of the detection unit (130) is embedded within the planar mat;
wherein the planar mat comprises a mattress (1024) positioned on the bed (1000) and forming the lying area of the bed (1000) so that the person leaving the bed (1000) generates the bed edge signal upon leaving the planar mat (1024);
wherein the detection unit (130) comprises a tubular probe (138), **characterized in that** the mattress (1024) comprises at least one transversal recess (1202) for folding the mattress (1024) and comprises at least one bridge element (1204, 1206) for bridging the tubular probe (138) over the at least one transversal recess (1202).

2. The device according to claim 1,
wherein the at least one bridge element (1204, 1206) comprises at least one of the group consisting of a stretchable guide element (1204) and a circumventing loop (1206) for circumventing the recess (1202).

3. The device according to claim 1 or any one of the above claims,
further comprising a signal duration determination unit (132) adapted for determining whether the bed edge signal is maintained for at least a predefined threshold duration;
wherein the trigger unit (134) is adapted for triggering an alarm in case the signal duration determination unit (132) has determined that the bed edge signal has maintained for at least the predefined threshold duration.

4. The device according to claim 1 or any one of the above claims,
wherein the detection unit (130) is adapted for detecting whether the bed edge signal has an amplitude of at least a predefined threshold amplitude;
wherein the trigger unit (134) is adapted for triggering an alarm in case the detection unit (130) has detected that the bed edge signal has an amplitude of at least the predefined threshold amplitude.

5. The device according to claim 1 or any one of the above claims,
wherein the detection unit (130) comprises a probe (138) having a lumen, particularly a lumen which comprises a fluid, and comprises a pressure sensor (140), particularly exactly one pressure sensor (140), adapted for measuring a pressure of a fluid within the lumen which fluid is impactable by an event of a person leaving a bed (1000).

6. The device according to claim 5,
wherein the probe (138) is adapted to be mountable on the bed (1000), particularly on at least one of the group consisting of the planar mat and a rail (106) of a bed (1000).

7. The device according to claim 1 or any one of the above claims,
wherein the planar mat comprises a mat body and a mat cover mounted over at least a part of the planar mat, wherein at least a part of the detection unit (130) is integrated in the mat cover.

8. A bed (1000), the bed (1000) comprising
a device according to claim 1 or any one of the above claims for generating an alarm upon detection of an event of a person leaving the bed (1000), the device being installed on the bed (1000).

9. A method of generating an alarm upon detection of an event of a person leaving a bed (1000), wherein the method comprises
detecting a bed edge signal indicative that a person has approached or left an edge of a bed (1000);
triggering an alarm in case the bed edge signal has been detected indicative that the person has approached or left the edge of the bed (1000);
providing a planar mat extending along an entire longitudinal extension of a lying area of the bed (1000), wherein at least a part of a detection unit (130) for detecting the bed edge signal is embedded within the planar mat;
wherein the planar mat comprises a mattress (1024) positioned on the bed (1000) and forming the lying area of the bed (1000) so that the person leaving the bed (1000) generates the bed edge signal upon leaving the planar mat;
wherein the detection unit (130) comprises a tubular probe (138), **characterized in that** the mattress (1024) comprises at least one transversal recess (1202) for folding the mattress (1024) and comprises at least one bridge element (1204, 1206) for bridging the tubular probe (138) over the at least one transversal recess (1202).

## Patentansprüche

1. Eine Vorrichtung zum Erzeugen eines Alarms als Folge einer Detektion von einem Ereignis, bei dem eine Person ein Bett (1000) verlässt, wobei die Vorrichtung aufweist
eine Detektionseinheit (130), welche eingerichtet ist zum Detektieren eines Bettkantensignals, welches indikativ dafür ist, dass sich eine Person an die Kante eines Bettes (1000) angenähert hat oder die Kante eines Bettes (1000) verlassen hat;
eine Triggereinheit (134), welche eingerichtet ist zum Triggern eines Alarms für den Fall, dass die Detektionseinheit (130) das Bettkantensignal detektiert hat, welches indikativ dafür ist, dass sich die Person an die Kante des Bettes (1000) angenähert hat oder die Kante des Bettes (1000) verlassen hat;
eine ebene Matte, welche sich entlang einer gesamten longitudinalen Erstreckung einer Liegefläche des Bettes (1000) erstreckt, wobei zumindest ein Teil der Detektionseinheit (130) innerhalb der ebenen Matte eingebettet ist; wobei die ebene Matte eine Matratze (1024) aufweist, welche auf dem Bett (1000) positioniert ist und welche die Liegefläche des Bettes (1000) bildet, so dass die Person, die das Bett (1000) verlässt, das Bettkantensignal erzeugt sobald sie die ebenen Matte (1024) verlässt;
wobei die Detektionseinheit (130) einen rohrförmigen Messgeber (138) aufweist, **dadurch gekennzeichnet, dass**
die Matratze (1024) zumindest eine transversale Aussparung (1202) zum Falten der Matratze (1024) aufweist und zumindest ein Brückenelement (1204, 1206) zum Überbrücken des rohrförmigen Messgebers (138) über die zumindest eine transversale Aussparung (1202) aufweist.

2. Die Vorrichtung gemäß Anspruch 1,
wobei das zumindest eine Brückenelement (1204, 1206) zumindest eines aus der Gruppe bestehend aus einem dehnbaren Führungselement (1204) und einer umgehenden Schlinge (1206) zum Umgehen der Aussparung (1202) aufweist.

3. Die Vorrichtung gemäß Anspruch 1 oder irgend einem der vorstehenden Ansprüche, ferner aufweisend
eine Signaldauer Bestimmungseinheit (132), welche eingerichtet ist zum Bestimmen, ob das Bettkantensignal für zumindest eine vorbestimmte Schwellen-Dauer aufrecht erhalten ist;
wobei die Triggereinheit (134) eingerichtet ist zum Triggern eines Alarms für den Fall, dass die Signaldauer Bestimmungseinheit (132) bestimmt hat, dass das Bettkantensignal für zumindest die vorbestimmte Schwellendauer aufrecht erhalten geblieben ist.

4. Die Vorrichtung gemäß Anspruch 1 oder irgend einem der vorstehenden Ansprüche, wobei
die Detektionseinheit (130) eingerichtet ist zum Detektieren, ob das Bettkantensignal eine Amplitude von zumindest einer vorbestimmten Schwellen-Amplitude hat; wobei
die Triggereinheit (134) eingerichtet ist zum Triggern eines Alarms für den Fall, dass die Detektionseinheit (130) bestimmt hat, dass das Bettkantensignal eine Amplitude von zumindest der vorbestimmten Schwellen-Amplitude hat.

5. Die Vorrichtung gemäß Anspruch 1 oder irgend einem der vorstehenden Ansprüche, wobei die Detektionseinheit (130)
einen Messgeber (138) aufweist, der ein Lumen hat, insbesondere ein Lumen, welches ein Fluid aufweist, und
einen Drucksensor (140), insbesondere genau einen einzigen Drucksensor(140), welcher eingerichtet ist zum Messen eines Drucks von einem Fluid innerhalb des Lumens, welches Fluid von einem Ereignis beeinflussbar ist, bei dem eine Person ein Bett (1000) verlässt.

6. Die Vorrichtung gemäß Anspruch 5, wobei
der Messgeber (138) eingerichtet ist um an dem Bett (1000) befestigt zu werden, insbesondere an zumindest einem aus der Gruppe bestehend aus der ebenen Matte und einer Schiene (106) von einem Bett (1000).

7. Die Vorrichtung gemäß Anspruch 1 oder irgend einem der vorstehenden Ansprüche, wobei
die ebene Matte einen Mattenkörper und eine Mattenabdeckung aufweist, welche über zumindest einem Teil der ebenen Matte angebracht ist, wobei zumindest ein Teil der Detektionseinheit (130) in der Mattenabdeckung integriert ist.

8. Ein Bett (1000), das Bett (1000) aufweisend
eine Vorrichtung gemäß Anspruch 1 oder irgend einem der vorstehenden Ansprüche zum Erzeugen eines Alarms als Folge einer Detektion von einem Ereignis, bei dem eine Person das Bett (1000) verlässt, wobei die Vorrichtung auf dem Bett (1000) installiert ist.

9. Ein Verfahren zum Erzeugen eines Alarms als Folge einer Detektion von einem Ereignis, bei dem eine Person ein Bett (1000) verlässt, wobei das Verfahren aufweist
Detektieren eines Bettkantensignals, welches indikativ dafür ist, dass sich eine Person an die Kante eines Bettes (1000) angenähert hat oder die Kante eines Bettes (1000) verlassen hat;
Triggern eines Alarms für den Fall, dass das Bettkantensignal detektiert worden ist, welches indikativ dafür ist, dass sich die Person an die Kante des Bettes (1000) angenähert hat oder die Kante des Bettes (1000) verlassen hat;
Bereitstellen einer ebenen Matte, welche sich entlang einer gesamten longitudinalen Erstreckung einer Liegefläche des Bettes (1000) erstreckt, wobei zumindest ein Teil von einer Detektionseinheit (130) zum Detektieren des Bettkantensignals innerhalb der ebenen Matte eingebettet ist;
wobei die ebene Matte eine Matratze (1024) aufweist, welche auf dem Bett (1000) positioniert ist und welche die Liegefläche des Bettes (1000) bildet, so dass die Person, die das Bett (1000) verlässt, das Bettkantensignal erzeugt sobald sie die ebenen Matte verlässt;
wobei die Detektionseinheit (130) einen rohrförmigen Messgeber (138) aufweist, **dadurch gekennzeichnet, dass**
die Matratze (1024) zumindest eine transversale Aussparung (1202) zum Falten der Matratze (1024) aufweist und zumindest ein Brückenelement (1204, 1206) zum Überbrücken des rohrförmigen Messgebers (138) über die zumindest eine transversale Aussparung (1202) aufweist.

## Revendications

1. Dispositif pour générer une alarme lors de la détection d'un événement d'une personne quittant un lit (1000), dans lequel le procédé comprend
une unité de détection (130) adaptée pour détecter un signal de bord de lit indicatif du fait qu'une personne s'est approchée ou a quitté un bord d'un lit (1000) ;
une unité de déclenchement (134) adaptée pour déclencher une alarme dans le cas où l'unité de détection (130) aurait détecté le signal de bord de lit indicatif du fait que la personne s'est approchée ou a quitté le bord du lit (1000) ;
une couche planaire s'étendant sur toute une extension longitudinale d'une zone de couchage du lit (1000), dans lequel au moins une partie de l'unité de détection (130) est incorporée dans la couche planaire ;
dans lequel la couche planaire comprend un matelas (1024) positionné sur le lit (1000) et formant la surface de couchage du lit (1000) de sorte que la personne quittant le lit (1000) génère le signal de bord de lit en quittant la couche planaire (1024) ;
dans lequel l'unité de détection (130) comprend une sonde tubulaire (138), **caractérisé en ce que** le matelas (1024) comprend au moins un évidement transversal (1202) pour plier le matelas (1024) et comprend au moins un élément de pont (1204, 1206) pour ponter la sonde tubulaire (138) sur l'au moins un évidement transversal (1202).

2. Dispositif selon la revendication 1,
dans lequel l'au moins un élément de pont (1204, 1206) comprend au moins un élément parmi le groupe comprenant un élément de guidage étirable (1204) et une boucle de contournement (1206) contournant l'évidement (1202).

3. Dispositif selon la revendication 1 ou l'une quelconque des revendications précédentes,
comprenant en outre une unité de détermination de durée de signal (132) adaptée pour déterminer si le signal de bord de lit est maintenu pendant au moins une durée de seuil prédéterminée ;
dans lequel l'unité de déclenchement (134) est adaptée pour déclencher une alarme dans le cas où l'unité de détermination de durée de signal (132) aurait déterminé que le signal de bord de lit s'est maintenu pendant au moins la durée de seuil prédéterminée.

4. Dispositif selon la revendication 1 ou l'une quelconque des revendications précédentes,
dans lequel l'unité de détection (130) est adaptée pour détecter si le signal de bord de lit a une amplitude d'au moins une amplitude de seuil prédéterminée ;
dans lequel l'unité de déclenchement (134) est adaptée pour déclencher une alarme dans le cas où l'unité de détection (130) aurait détecté que le signal de bord de lit a une amplitude d'au moins l'amplitude de seuil prédéterminée.

5. Dispositif selon la revendication 1 ou l'une quelconque des revendications précédentes,
dans lequel l'unité de détection (130) comprend une sonde (138) ayant une lumière, en particulier une lumière qui comprend un fluide, et comprend un capteur de pression (140), en particulier exactement un capteur de pression (140), adapté pour mesurer une pression d'un fluide à l'intérieur de la lumière, lequel fluide peut être pressé par un événement d'une personne quittant le lit (1000).

6. Dispositif selon la revendication 5,
dans lequel la sonde (138) est adaptée pour pouvoir être montée sur le lit (1000), en particulier au moins un élément parmi le groupe comprenant la couche planaire et un rail (106) d'un lit (1000).

7. Dispositif selon la revendication 1 ou l'une quelconque des revendications précédentes,
dans lequel la couche planaire comprend un corps de couche et une couverture de couche installée sur au moins une partie de la couche planaire, dans lequel au moins une partie de l'unité de détection (130) est intégrée dans la couverture de couche.

8. Lit (1000), le lit (1000) comprenant
un dispositif selon la revendication 1 ou l'une quelconque des revendications précédentes pour générer une alarme lors de la détection d'un événement d'une personne quittant le lit (1000), le dispositif étant installé sur le lit (1000).

9. Procédé pour générer une alarme lors de la détection d'un événement d'une personne quittant un lit (1000), dans lequel le procédé comprend
la détection d'un signal de bord de lit indicatif du fait qu'une personne s'est approchée ou a quitté un bord d'un lit (1000) ;
le déclenchement d'une alarme dans le cas où le signal de bord de lit aurait été détecté, indicatif du fait que la personne s'est approchée ou a quitté le bord du lit (1000) ;
la prédisposition d'une couche planaire s'étendant sur toute l'extension longitudinale d'une zone de couchage du lit (1000), dans lequel au moins une partie d'une unité de détection (130) pour détecter le signal de bord de lit est incorporée dans la couche planaire ;
dans lequel la couche planaire comprend un matelas (1024) positionné sur le lit (1000) et formant la surface de couchage du lit (1000) de sorte que la personne quittant le lit (1000) génère le signal de bord de lit en quittant la couche planaire ;
dans lequel l'unité de détection (130) comprend une sonde tubulaire (138), **caractérisé en ce que** le matelas (1024) comprend au moins un évidement transversal (1202) pour plier le matelas (1024) et comprend au moins un élément de pont (1204, 1206) pour ponter la sonde tubulaire (138) sur l'au moins un évidement transversal (1202).
